# EUROPÄISCHE PATENTSCHRIFT

(11) **EP 1 850 802 B1**
(45) Veröffentlichungstag und Bekanntmachung des Hinweises auf die Patenterteilung: **11.04.2012**
(21) Anmeldenummer: 06707303.1
(22) Anmeldetag: 27.02.2006
(51) Int. Cl.: A61F 2/36, C22F 1/18, C22C 14/00

(54) **VERFAHREN ZUM HERSTELLEN EINES MEDIZINISCHEN IMPLANTATS AUS EINER BETA-TITAN-MOLYBDÄN-LEGIERUNG UND ENTSPRECHENDES IMPLANTAT**
METHOD OF PRODUCING A MEDICAL IMPLANT MADE OF A BETA-TITANIUM-MOLYBDENUM-ALLOY AND ACCORDING IMPLANT
PROCÉDÉ DE FABRIQUER UN IMPLANT MEDICAL D'UN ALLIAGE DE BETA-TITANE ET MOLYBDÈNE ET UN IMPLANT CORRESPONDANT

(30) Priorität: 25.02.2005 EP 05004180
(43) Veröffentlichungstag der Anmeldung: 07.11.2007
(73) Patentinhaber: WALDEMAR LINK GmbH & Co. KG, 22339 Hamburg (DE)
(72) Erfinder: BALIKTAY, Sevki, 14050 Berlin (DE); KELLER, Arnold, 23863 Kayhude (DE)
(74) Vertreter: Glawe, Delfs, Moll
(86) Internationale Anmeldenummer: PCT/EP2006/001792
(87) Internationale Veröffentlichungsnummer: WO 2006/089792

(56) Entgegenhaltungen:
- US-A1- 2004 136 859
- US-A1- 2004 168 751
- US-B1- 6 238 491
- DONACHIE ET AL: "Titanium - a technical guide" Dezember 2002 (2002-12), ASM INTERNATIONAL , USA , XP002336954 ISBN: 0-87170-686-5 Seite 39 - Seite 42; Abbildung 6.5

## Beschreibung

Die Erfindung betrifft ein Verfahren zum Herstellen eines medizinischen Implantats aus einer Titanlegierung sowie ein entsprechendes Implantat.

Als Material zur Herstellung von Implantaten finden Titanlegierungen immer breitere Verwendung. Zu den Vorzügen dieses Materials zählen für den Einsatz als Prothese so wichtige Eigenschaften wie eine hohe mechanische Belastbarkeit, eine hohe chemische Beständigkeit und nicht zuletzt hervorragende Biokompatibilität. Unter dem Gesichtspunkt der Materialeigenschaften sind Titanlegierungen das Material der Wahl für viele verschiedene Arten von Implantaten, darunter Knochenplatten, Stifte, künstliche Knie- und Hüftgelenke sowie Bandscheibenprothesen.

Im Stand der Technik sind unterschiedliche Verfahren zur Herstellung der Implantate bekannt. Die Auswahl eines geeigneten Verfahrens richtet sich nicht nur nach der herzustellenden Art des Implantats, sondern auch nach der jeweils verwendeten Titanlegierung. Zur Formgebung sind es im Wesentlichen zwei Verfahren, die für Titanlegierungen verwendet werden. Das sind Schmieden einerseits und Feingießen andererseits. Im Grunde handelt es sich bei Titanlegierungen um Schmiedelegierungen (Peters/Leyens: Titan und Titanlegierungen, Wiley-VCH-Verlag, 2002). Das Feingießen hat aber den Vorteil, dass sich damit auch komplexe Formen leicht herstellen lassen, die durch Schmieden nicht oder nur unter Fügung mehrerer Komponenten zu erreichen sind. Das Feingießen von Titanlegierungen ist jedoch wegen des hohen Schmelzpunkts und der großen Reaktionsfreudigkeit des Titans generell problematisch; dazu kommt die geringe Dichte der Legierungen. Nur einige Gruppen von Titanlegierungen eignen sich zum Feingießen. Dazu zählen insbesondere sog. α-Titanlegierungen und einige α/β-Titanlegierungen. Aus letztgenannter Gruppe haben insbesondere Legierungen mit Vanadium und Aluminium, wie TiAl6V4, Bedeutung für die Herstellung von Implantaten erlangt. Durch Feinguss können aus dieser Legierung Implantate, wie Gelenkprothesen oder Dentalimplantate, mit gutem Erfolg hergestellt werden.

Allerdings bestehen gewisse Bedenken in Bezug auf die langfristige Verträglichkeit der Legierungselemente, wie sie typischerweise für α/β-Titanlegierungen wie TiAl6V4 verwendet werden. Außerdem liegt der Elastizitätsmodul dieser Legierungen beträchtlich über dem des natürlichen Knochenmaterials, wodurch es zu pathologischen Veränderungen des Knochens kommen kann.

Aus US 2004/0136859 A1 ist es bekannt, dass medizinische Implantate aus β-Titanlegierungen gegossen werden können.

Der Erfindung liegt die Aufgabe zugrunde, medizinische Implantate zu schaffen, bei denen diese Nachteile vermindert sind.

Die erfindungsgemäße Lösung liegt in den Merkmalen der unabhängigen Ansprüche. Vorteilhafte Weiterbildungen sind Gegenstand der abhängigen Ansprüche.

Es ist ein Verfahren zum Herstellen eines medizinischen Implantats aus einer Titanlegierung vorgesehen, umfassend die Schritte Feingießen der Titanlegierung in einer dem herzustellenden Implantat entsprechenden Gussform, wobei erfindungsgemäß vorgesehen ist, dass eine β-Titanlegierung verwendet wird, heißisostatisch gepresst, lösungsgeglüht und anschließendes abgeschreckt wird. Die β-Titanlegierung hat einen Molybdängehalt von 7,5 % - 25 % Molybdän, der Rest ist Titan. Der Schritt des heißisostatischen Pressens wird bei einer Temperatur durchgeführt, die zwischen der Beta-Transustemperatur und 100 °C unterhalb der Beta-Transustemperatur liegt. Für das Lösungsglühen wird eine Temperatur zwischen 700 °C und 900 °C gewählt.

Mit dem erfindungsgemäßen Verfahren wird es ermöglicht, medizinische Implantate aus einer β-Titanlegierung im Feinguss herzustellen. Die Möglichkeit, β-Titanlegierungen zu verwenden, bringt in Bezug auf medizinische Implantate beachtliche Vorteile mit sich. So weisen β-Titanlegierung günstige mechanische Eigenschaften auf, insbesondere einen deutlich niedrigeren Elastizitätsmodul als die bekannten α/β-Titanlegierungen. Während letztere üblicherweise Werte von ca. 100.000 N/mm² aufweisen, können beispielsweise mit Titan-Molybdänlegierungen beinahe um die Hälfte verminderte Werte von ca. 60.000 N/mm² erreicht werden. Weiterhin kann durch die Verwendung von β-Titanlegierungen die Biokompatibilität erhöht werden. Während bei der häufig verwendeten α/β-Titanlegierung TiAl6V4 gewisse Bedenken im Hinblick auf eine Toxizität freigesetzter Aluminium- oder Vanadium-Ionen bestehen, können für β-Titanlegierung in Bezug auf Toxizität unbedenkliche Legierungsbildner wie Molybdän mit Erfolg verwendet werden. Es hat sich gezeigt, dass insbesondere mit Titan-Molybdänlegierungen hervorragende Ergebnisse in Bezug auf die mechanischen Eigenschaften wie auch in Bezug auf Biokompatibilät erreicht werden. Der Molybdängehalt beträgt zwischen 7,5 und 25 %, vorzugsweise zwischen 12 und 16 %. Damit kann durch schnelles Abkühlen nach dem Gießen eine metastabile β-Phase erreicht werden. Besonders bewährt hat sich TiMo15 mit einem Molybdängehalt von 15 %.

Weiter hat die Verwendung von β-Titanlegierung den Vorteil, dass auch Implantate mit komplexer Form rationell hergestellt werden können. Generell ist Feinguss ein Formgebungsverfahren, mit dem im Vergleich zur Formgebung durch Schmieden auch komplexe Formen rationell herstellbar sind. Aber es hat sich gezeigt, dass gerade bei der Verwendung mit den aus dem Stand der Technik bekannten α- oder α/β-Titanlegierungen es nur unzureichend gelang, beim Feinguss scharfe Kanten an den Implantaten zu erzeugen. Die an sich gegebenen Vorteile des Feingießens, nämlich beliebige, komplexe Formen erzeugen zu können, konnten so nicht voll realisiert werden. Gerade bei Implantaten ist es aber häufig wünschenswert, zur besseren Verankerung des Implantats scharfe Kanten vorzusehen. Für eine zementlose Implantation, der aus Gründen der langfristigen mechanischen Stabilität des Implantats in vielen Fällen der Vorzug zu geben ist, sind scharfe Kanten an den Implantaten von großer Bedeutung. Überraschenderweise hat sich gezeigt, dass das erfindungsgemäße Verfahren eine verbesserte Formfüllung erreicht. Damit können auch bei komplex geformten Implantaten mit hoher Qualität scharfe Kanten erreicht werden. Damit ermöglicht die Erfindung nicht nur Implantate mit günstigeren Eigenschaften in Bezug auf Mechanik und Biokompatibilität, sondern auch in Bezug auf eine Verbesserung der Formgebung durch den Feinguss.

Zweckmäßigerweise ist beim Lösungsglühen der Temperaturverlauf so gewählt, dass die Titanlegierung frei von ω-Phasen ist. Der Gefahr einer Beeinträchtigung der mechanischen Eigenschaften durch Bildung einer ω-Phase wird damit entgegengewirkt.

Für das heißisostatische Pressen (HIP) ist eine Temperatur unterhalb der β-Transustemperatur günstig, und zwar bis zu 100 °C darunter. Für eine Titanmolybdänlegierung mit 15 % Molybdänanteil haben sich Temperaturen im Bereich von 710 °C bis 760 °C, vorzugsweise von etwa 740 °C, bei einem Argondruck von etwa 1100 bis 1200 bar bewährt.

Für das Lösungsglühen haben sich Temperaturen von mindestens 700 °C bis zu 880 °C bewährt, vorzugsweise im Bereich von 800 °C bis 860 °C unter einer Argonschutzgasatmosphäre. Damit wird eine Verbesserung der Duktilität der Legierung erreicht. Eine Vorauslagerung vor oder nach dem heißisostatischen Pressen ist dabei nicht erforderlich. Das anschließende Abschrecken erfolgt vorzugsweise mit kaltem Wasser.

Es kann von Vorteil sein, den Gegenstand zum Abschluss noch zu härten. Hiermit kann bei Bedarf der Elastizitätsmodul noch erhöht werden. Vorzugsweise geschieht dazu das Härten in einem Temperaturbereich von ca. 600 °C bis ca. 700 °C.

Die Erfindung bezieht sich weiter auf ein gemäß dem oben genannten Verfahren hergestelltes medizinisches Implantat sowie auf eines gemäß dem nebengeordneten Anspruch. Danach wird ein medizinisches Implantat aus einer feingegossenen β-Titan-Molybdänlegierung mit einem Molybdängehalt von 15 %, Rest Titan bereitgestellt. Das Implantat hat eine Korngröße von 0,3 mm und einen Elastizitätsmodul zwischen 59,4 kN/mm² und 68 kN/mm². Zur näheren Erläuterung wird auf obige Ausführungen verweisen.

Ergänzt sei noch Folgendes: Bei dem Implantat kann es sich um eine orthopädische Prothese, vorzugsweise um eine Gelenkprothese, handeln. Gelenkprothesen sind statisch und dynamisch hoch beansprucht. Besondere Bedeutung kommt der Lastüberleitung in die umgebende Knochenstruktur zu. Sie soll möglichst physiologisch günstig sein. Kritisch ist dabei, dass eine ungünstige Lastüberleitung von der Prothese zum umgebenden Knochen zu einer Degeneration des Knochengewebes führen kann. Das hat nicht selten eine Lockerung der Prothese zur Folge. Untersuchungen haben gezeigt, dass Prothesen aus einem Material mit niedrigerem Elastizitätsmodul eine Belastungssituation erzeugen, die physiologischer ist als bei Prothesen aus steifem Material. Das gilt insbesondere für Prothesen mit langen Schäften, wie das Femurteil einer Hüftprothese oder andere Gelenkprothesen. Der Elastizitätsmodul liegt bei einer herkömmlichen Titanlegierung, zum Beispiel TiAl6V4, bei ca. 100.000 N/mm² und damit beträchtlich über dem Elastizitätsmodul des kortikalen Knochenmaterials von etwa 25.000 N/mm². Mit dem erfindungsgemäßen Implantat können niedrigere Werte erreicht werden. So weist ein erfindungsgemäß hergestelltes Implantat aus TiMo15 einen Elastizitätsmodul von ca. 60.000 N/mm² auf, also nur etwas mehr als die Hälfte des Werts von TiAl6V4. Das ist insbesondere für Gelenkprothesen mit langen Schäften, wie Hüft-, Knie-, Schulter- oder Ellenbogenprothesen ein großer Vorteil. Denn damit ergibt sich eine wesentliche Verbesserung in Bezug auf die Kraftüberleitung zum Knochen.

Entsprechende Erwägungen gelten für eine Ausführung des erfindungsgemäßen Implantats als Dentalprothese.

Die Erfindung wird nachfolgend unter Bezugnahme auf die Zeichnung erläutert, in der ein vorteilhaftes Ausführungsbeispiel dargestellt ist. Es zeigen:
- Fig. 1: eine Abbildung des Mikrogefüges in einem Gusszustand unmittelbar nach dem Giessen;
- Fig. 2: eine Abbildung des Mikrogefüges nach dem Hippen;
- Fig. 3: eine Abbildung des Mikrogefüges nach dem Lösungsglühen mit anschließendem Abschrecken;
- Fig. 4: eine Ansicht eines Femurteils eines Ausführungsbeispiels für ein erfindungsgemäßes Implantat;
- Fig. 5: eine Ansicht eines Dentalimplantats als weiteres Ausführungsbeispiel; und
- Fig. 6: eine Tabelle mit mechanischen Eigenschaften der erfindungsgemäß verarbeiteten Titanlegierung.

Zuerst sei ein Weg zur Durchführung des erfindungsgemäßen Verfahrens beschrieben. Das hergestellte Implantat wird später am Beispiel eines Femurteils einer Hüftprothese erläutert.

Ausgangsmaterial ist eine β-Titanlegierung mit einem Molybdänanteil von 15 % (TiMo15). Diese Legierung kann handelsüblich in Form von kleinen Barren (Ingots) erworben werden.

In einem ersten Schritt erfolgt ein Feinguss der zu gießenden Gegenstände. Unter Gegenstand wird vorliegend ein zur Endverwendung geformtes Implantat, wie Endoprothesen, z. B. Hüftprothesen oder andere Gelenkprothesen, oder unbewegliche Implantate, beispielsweise Platten und Stifte oder Dentalimplantate, verstanden. Nicht umfasst sind Barren, die zur Weiterverarbeitung durch Umformverfahren gedacht sind, also insbesondere nicht durch Kokillenguss hergestellte Ingots zur Weiterverarbeitung durch Schmieden oder andere Formgebungsverfahren.

Zum Schmelzen und Gießen des TiMo15 ist eine Gießanlage vorgesehen. Vorzugsweise handelt es sich um eine Kaltwandtiegel-Vakuuminduktions-Schmelz- und Gießanlage. Mit einer solchen Anlage können die hohen Temperaturen, die für ein sicheres Schmelzen von TiMo15 zum Feingießen erforderlich sind, erreicht werden. Der Schmelzpunkt von TiMo15 liegt bei 1770 °C zuzüglich eines Zuschlags von ca. 60 °C für ein sicheres Feingießen. Insgesamt muss also eine Temperatur von 1830 °C erreicht werden. Das Feingießen der Schmelze erfolgt anschließend mittels an sich bekannter Verfahren, beispielsweise mit Wachskernen und Keramikformen als verlorene Form. Derartige Feingusstechniken sind zum Feingießen von TiAl6V4 bekannt.

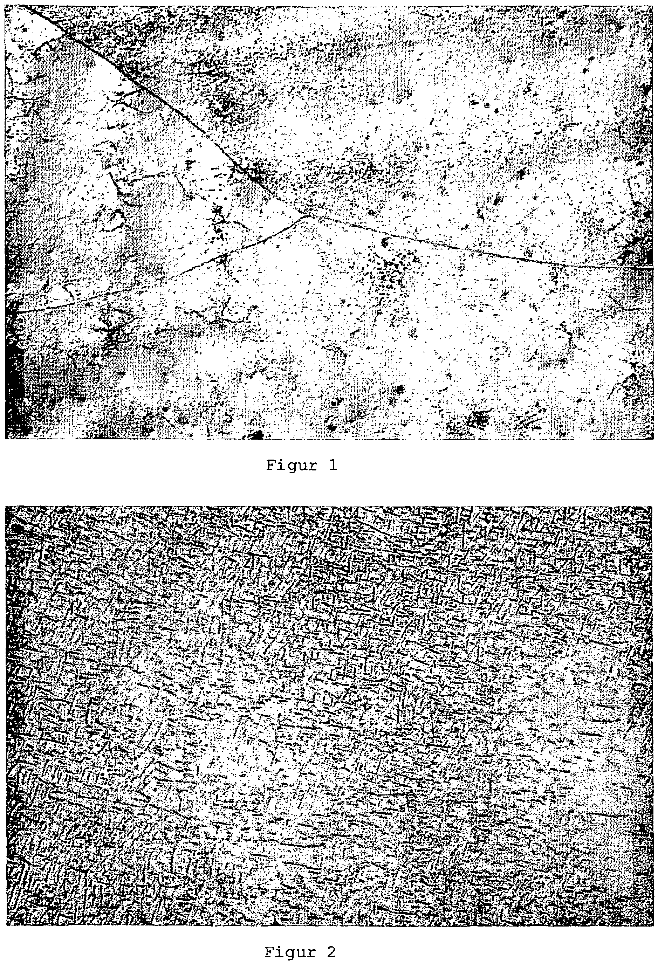

Wie man an der Abbildung (1000fache Vergrößerung) in Fig. 1 erkennen kann, bilden sich Dendriten und in interdendritischen Zonen zeigen sich erhebliche Ausscheidungen. Dies ist eine Folge der so genannten negativen Seigerung von Titan-Molybdänlegierungen. Dieser Effekt beruht auf dem speziellen Verlauf der Liquidus- und Solidustemperatur bei Titan-Molybdänlegierungen. In der Schmelze erstarren zuerst die Bereiche mit hohem Molybdänanteil, wobei sich die in der Abbildung zu erkennenden Dendriten bilden. Als Folge davon verarmt die Restschmelze, d. h. ihr Molybdängehalt sinkt. Die interdendritischen Zonen haben im Gussgefüge einen Molybdängehalt von unter 15 %, der sogar auf Werte von ca. 10 % absinken kann. Als Folge der Molybdänverarmung fehlt in den interdendritischen Zonen eine ausreichende Menge an β-Stabilisatoren. Das hat zur Folge, dass sich lokal eine erhöhte a/β-Umwandelungstemperatur einstellt, wodurch die in Fig. 1 gut zu erkennenden Ausscheidungen entstehen.

Es ist zweckmäßig, eine beim Giessen eventuell entstandene Randzone in Gestalt einer harten, spröden Schicht (sog. α-case) durch Beizen zu entfernen. Üblicherweise weist diese Schicht eine Dicke von ca. 0,03 mm auf.

Um den ungünstigen Effekt der negativen Seigerung mit den Ausscheidungen in den interdendritischen Zonen entgegenzuwirken, werden die nach dem Feingießen von den Gießformen befreiten Gusskörper erfindungsgemäß einer Wärmebehandlung unterzogen. Dazu ist ein heißisostatisches Pressen (HIP) vorgesehen, und zwar bei einer Temperatur knapp unterhalb der β-Transustemperatur. Sie kann im Bereich 710 °C bis 760 °C liegen, vorzugsweise beträgt sie etwa 740 °C. Das Pressen erfolgt durch Argon mit einem Druck von 1100 bis 1200 bar. Dabei gehen die unerwünschten Ausscheidungen in den interdendritischen Zonen wieder in Lösung. Allerdings scheiden sich bei der Abkühlung nach dem Hippen wiederum feine sekundäre Phasen aus, und zwar bevorzugt in den ursprünglichen interdendritischen Zonen (siehe Fig. 2, 1000fache Vergrößerung). Das hat eine unerwünschte Versprödung des Materials zur Folge.

Aus diesem Grund weisen die Gegenstände nach dem Hippen eine nur geringe Duktilität auf.

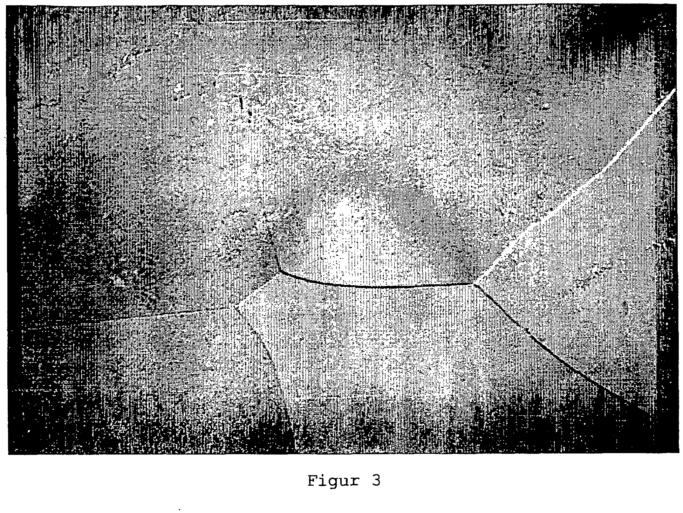

Um die störenden Ausscheidungen zu beseitigen, werden die Gusskörper in einem Kammerofen unter Argon-Schutzgasatmosphäre geglüht. Dazu wird ein Temperaturbereich von ca. 700 °C bis 860 °C gewählt, bei einer Dauer von mehreren, meist zwei Stunden. Es besteht hierbei ein gegenläufiger Zusammenhang zwischen der Temperatur und der Dauer, bei höherer Temperatur genügt eine kürzere Zeit und umgekehrt. Nach dem Lösungsglühen werden die Gusskörper mit kaltem Wasser abgeschreckt. In Fig. 3 (1000fache Vergrößerung) ist das Gefüge nach dem Lösungsglühen dargestellt. Man erkennt primäre β-Körner und innerhalb der Körner sehr feine interdendritisch angeordnete Ausscheidungen (siehe wolkenartige Ansammlung links oben in der Abbildung). Die mit dem erfindungsgemäßen Verfahren feingegossenen Gegenstände weisen in ihrer Kristallstruktur β-Körner mit einer mittleren Größe von über 0,3 mm auf. Diese Größe ist typisch für die mit dem erfindungsgemäßen Verfahren erreichte Kristallstruktur.

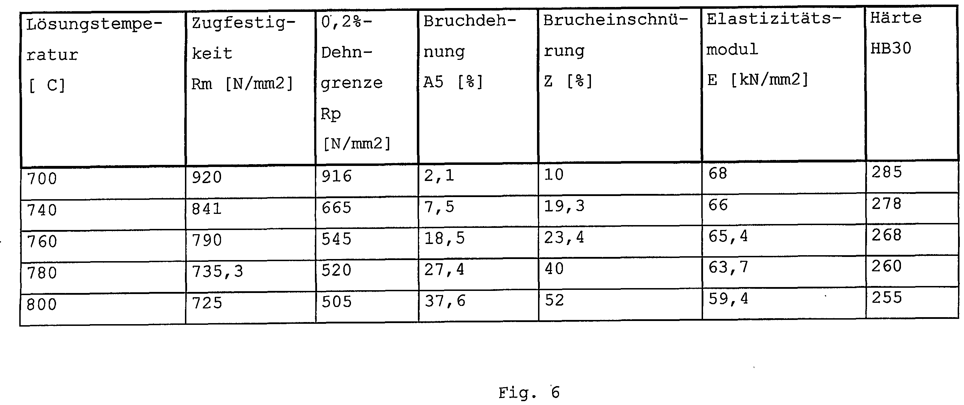

Die nach dem Lösungsglühen erreichten mechanischen Eigenschaften sind in der Tabelle in Fig. 6 wiedergegeben.

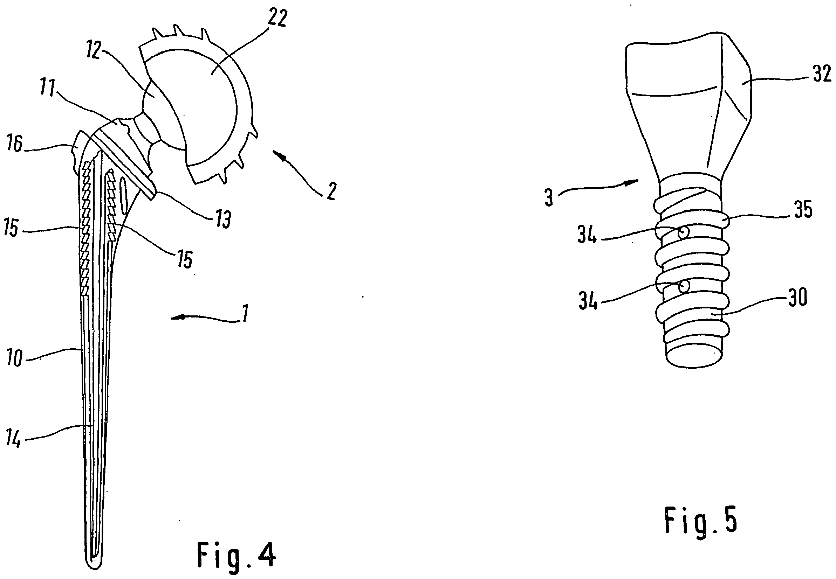

Das in Fig. 4 dargestellte Ausführungsbeispiel zeigt ein Femurteil 1 einer Hüftprothese. Es besteht aus einer β-Titanlegierung, nämlich TiMo15. Sie weist bei Raumtemperatur eine mittenzentrierte kubische Kristallstruktur auf.

Das Femurteil 1 ist zur Implantation am oberen Ende des Femur vorgesehen. Er weist einen lang gestreckten Schaft 10 und einen sich stumpfwinklig anschließenden Hals 11 auf. An seinem schaftfernen Ende ist ein Gelenkkopf 12 angeordnet, der mit einem Acetabulumteil 2 ein Gelenk bildet. Zur Implantation erfolgt eine vollständige oder teilweise Resektion des Oberschenkelhalses mit seinem Kopf, und ein Zugang zu der Markhöhle des Femur wird eröffnet. Über diesen Zugang wird der Schaft 10 des Femurteils 1 in die Markhöhle eingeführt und dort verankert. Je nach Ausführung ist Zement als Verankerungsmittel vorgesehen, oder die Fixierung erfolgt zementlos.

Das Femurteil 1 leitet auf die Hüftprothese einwirkenden mechanischen Belastungen, seien es statische beim Stehen oder dynamische beim Laufen, in den Femur ein. Für eine dauerhafte sichere Verankerung des Femurteils 1 im Knochenmaterial des Femur ist eine physiologisch günstige Belastungsübertragung wichtig. Ist das Femurteil 1 steif ausgeführt, übernimmt es einen Großteil der Belastung und entlastet somit das Knochenmaterial insbesondere im oberen Bereich des Femur. Das führt auf längere Sicht zu einer Degeneration des Femur in diesem Bereich. Als Folge droht eine Lockerung des Femurteils 1, schließlich ein Ausfall der Prothese. Um diesen Ausfallmodus zu verhindern, ist es günstig, das Femurteil 1 weniger steif auszuführen. Insbesondere der Schaft 10 des Femurteils 1 ist insoweit kritisch. Das Knochenmaterial des Femur weist in dem kortikalen Bereich einen Elastizitätsmodul von ca. 20.000 bis 25.000 N/mm² auf. Das Femurteil 1 weist erfindungsgemäß einen Elastizitätsmodul von lediglich ca. 60.000 N/mm² auf. Herkömmlicherweise verwendete Materialien wie TiA16V4 weisen einen Elastizitätsmodul von ca. 100.000 N/mm² oder gar 200.000 N/mm² bei Kobalt-Chromlegierungen auf. Das erfindungsgemäße Das Femurteil 1 weist also einen physiologisch günstigen niedrigen Elastizitätsmodul auf. Besonders im Bereich des in dieser Hinsicht kritischen Schafts 10 ist der niedrigere Elastizitätsmodul ein großer Vorteil für die Langzeitverträglichkeit der Prothese.

Dank der Erfindung können im Feinguss auch komplexe Formen leicht hergestellt werden. So weist das Femurteil 1 an seinem Schaft 10 eine Vielzahl von Ausnehmungen und sägezahnartigen Vorsprüngen auf. Sie dienen einer verbesserten Verankerung des Femurteils 1 in dem Femur, und ermöglichen eine zementlose Implantation. Man erkennt mehrere in Längsrichtung des Schafts 10 verlaufende Rillen 14. Sie sind sowohl auf der anterioren und posterioren Seite des Schafts 10 angeordnet, können aber auch an den Lateralseiten vorgesehen sein. Im oberen Bereich des Schafts 10 sind mehrere Reihen mit Sägezahnvorsprüngen 15 vorgesehen. Weiter ist im Übergang zum Hals 11 ein umlaufender Ring 13 vorgesehen. Er kann als gesondertes Element ausgeführt sein, dank der Erfindung kann er aber auch einstückig mit dem Schaft 10 und Hals 11 sein. Derart komplexe Formen aufweisende Prothesenteile können herkömmlicherweise nur aus TiAl6V4 hergestellt werden. Dieses weist aber - wie oben bereits erläutert - einen unerwünscht hohen Elastizitätsmodul auf. Zwar ist es auch bekannt, Femurteile aus β-Titanlegierungen herzustellen, dann aber nur im Schmiedeverfahren. Durch Schmieden können so komplexe und aus medizinischer Sicht vorteilhafte Formen, wie sie in Fig. 4 dargestellt sind, nicht hergestellt werden. Es ist das Verdienst der Erfindung, dass solch komplexe Formen auch bei Hüftprothesen aus β-Titanlegierungen erreicht werden können.

Als ein weiteres Ausführungsbeispiel ist ein Dentalimplantat in Fig. 5 dargestellt. Ein solches Dentalimplantat 3 hat die Funktion eines künstlichen Fundaments. Es soll die natürliche Zahnwurzel ersetzen und dient der Befestigung von Zahnprothesen (nicht dargestellt) an seinem Kopf 32. Das Dentalimplantat 3 muss in erster Linie zwei verschiedene Bedingungen erfüllen. Es muss einerseits einer hohen Belastung standhalten. Auf einen Zahn können beim Kauen statische Kräfte bis zu 550 N einwirken. Diese Kräfte muss das Dentalimplantat über Jahre hinweg als Wechselbelastung aufnehmen können, und in den Kieferknochen einleiten können. Das führt zu der zweiten Bedingung, nämlich der Gewährleistung einer guten Knocheneinbindung. Die Einleitung der beim Kauen auftretenden Kräfte ist nicht unproblematisch, zumal Dentalimplantate nur einen recht dünnen Schaft 30 aufweisen. Um eine optimale Verankerung in dem Kieferknochen zu erreichen und damit das Risiko einer Lockerung entgegenzuwirken, ist der Schaft 30 mit einem Schraubengewinde 35 versehen. Zur weiteren Verbesserung der Verankerung können Queröffnungen 34 vorzugsweise als Durchgangsöffnungen vorgesehen sein. Sie begünstigen ein Einwachsen des Dentalimplantats in den Kieferknochen und sind damit für eine sichere und dauerhafte Verankerung sehr förderlich, jedoch führen sie zu Spannungsspitzen und damit zu einer höheren mechanischen Belastung des Schafts 30. Dank des erfindungsgemäßen Verfahrens können derartige Dentalimplantate 3 im Feingussverfahren aus β-Titanlegierungen hergestellt werden. Dabei können auch komplexe Formen, wie das Gewinde 35 und die Queröffnungen 34 rationell erzeugt werden, ohne dass eine aufwendige Nachbearbeitung, beispielsweise durch spanabhebende Bearbeitung erforderlich ist. Es braucht damit bei der Wahl einer geeigneten Titanlegierung und bei der Auslegung sowie Dimensionierung keine Rücksicht auf die mechanische Bearbeitbarkeit genommen zu werden. Es lassen sich damit Gestaltungen realisieren, die mit herkömmlicher Formgebung durch Schmieden oder mechanische Bearbeitung praktisch nicht zu realisieren waren. Zudem gilt auch für Dentalimplantate das vorstehend zu der Femurprothese ausgeführte, dass dank des geringen Elastizitätsmoduls die Gefahr einer Degeneration der umgebenden Knochenstruktur minimiert ist.

## Patentansprüche

1. Verfahren zum Herstellen eines medizinischen Implantats aus einer Titanlegierung, umfassend die Schritte:
Feingießen der Titanlegierung in einer dem herzustellenden Implantat entsprechenden Gussform,
**gekennzeichnet durch**:
Verwenden einer β-Titanlegierung mit einem Molybdängehalt von 7,5% bis 25%, Rest Titan;,
heißisostatisches Pressen bei einer Temperatur, die maximal so hoch ist wie die Beta-Transustemperatur der Titan-Molybdänlegierung und minimal 100 °C unter der Beta-Transustemperatur liegt,
Lösungsglühen bei einer Temperatur zwischen 700°C und 900°C, und
anschließendes Abschrecken.

2. Verfahren nach Anspruch 1,
**gekennzeichnet durch**
Härten nach dem Abschrecken.

3. Verfahren nach Anspruch 1 oder 2,
**dadurch gekennzeichnet, dass**
die Titan-Molybdänlegierung einen Molybdänanteil von etwa 15 % aufweist.

4. Verfahren nach einem der vorhergehenden Ansprüche,
**dadurch gekennzeichnet, dass**
das Abschrecken mit vorzugsweise kaltem Wasser erfolgt.

5. Medizinisches Implantat, aus einer feingegossenen β-Titan-Molybdänlegierung mit einem Molybdänanteil von 15%, Rest Titan, das eine mittlere Korngröße von mindestens 0,3 mm und einen Elastizitätsmodul von 59,4 kN/mm² bis 68 kN/mm² aufweist.

6. Implantat nach Anspruch 5,
**dadurch gekennzeichnet, dass**
es heißisostatisch gepresst und lösungsgeglüht ist.

7. Implantat nach Anspruch 5 oder 6;
**dadurch gekennzeichnet, dass**
die Titanlegierung frei von einer ω-Phase ist.

8. Implantat nach einem der Ansprüche 5 bis 7;
**dadurch gekennzeichnet, dass**
die Titanlegierung vanadium- und aluminiumfrei ist.

9. Implantat nach einem der Ansprüche 5 bis 8;
**dadurch gekennzeichnet, dass**
es eine orthopädische Prothese ist.

10. Implantat nach Anspruch 9,
**dadurch gekennzeichnet, dass**
die Prothese eine Gelenkprothese ist.

11. Implantat nach Anspruch 10,
**dadurch gekennzeichnet, dass**
es ein Femurteil (1) einer Hüftgelenkprothese ist.

12. Implantat nach einem der Ansprüche 5 bis 8,
**dadurch gekennzeichnet, dass**
es ein Dentalimplantat (3) ist.

## Claims

1. Process for producing a medical implant from a titanium alloy, comprising the steps of:
investment-casting the titanium alloy in a casting mold which corresponds to the implant that is to be produced,
**characterized by**:
using a β-titanium alloy having a molybdenum content of from 7.5% to 25%, remainder titanium;
hot isostatic pressing at a temperature which is at most equal to a beta-transus temperature of the titanium-molybdenum alloy and is at most 100°C below the beta-transus temperature,
solution annealing at a temperature between 700°C and 900°C, and
then quenching.

2. Process according to Claim 1,
**characterized by**
hardening following the quenching.

3. Process according to Claim 1 or 2,
**characterized in that**
the titanium-molybdenum alloy has a molybdenum content of approximately 15%.

4. Process according to one of the preceding claims,
**characterized in that**
the quenching is carried out using preferably cold water.

5. Medical implant made from an investment-cast β-titanium-molybdenum alloy having a molybdenum content of 15%, remainder titanium, which has a mean grain size of at least 0.3 mm and a modulus of elasticity of from 59.4 kN/mm² to 68 kN/mm².

6. Implant according to Claim 5,
**characterized in that**
it is hot-isostatically pressed and solution-annealed.

7. Implant according to Claim 5 to 6,
**characterized in that**
the titanium alloy is free of a ω-phase.

8. Implant according to one of Claims 5 to 7,
**characterized in that**
the titanium alloy is free of vanadium and aluminum.

9. Implant according to one of Claims 5 to 8,
**characterized in that**
it is an orthopedic prosthesis.

10. Implant according to Claim 9,
**characterized in that**
the prosthesis is a joint prosthesis.

11. Implant according to Claim 10,
**characterized in that**
it is a femur part (1) of a hip joint prosthesis.

12. Implant according to one of Claims 5 to 8,
**characterized in that**
it is a dental implant (3).

## Revendications

1. Procédé de fabrication d'un implant médical en un alliage de titane, le procédé comportant les étapes qui consistent à :
couler à modèle perdu l'alliage de titane dans un moule de coulée qui correspond à l'implant à fabriquer, **caractérisé par**
l'utilisation d'un alliage de titane β dont la teneur en molybdène est de 7,5 à 25 %, le reste étant du titane,
la compression isostatique à chaud à une température au plus aussi élevée que la température de transition beta de l'alliage de titane et de molybdène et d'au moins 100°C plus basse que la température de transition beta,
un recuit de solubilisation à une température comprise entre 700°C et 900°C et ensuite
une trempe.

2. Procédé selon la revendication 1, **caractérisé par** un durcissement réalisé après la trempe.

3. Procédé selon les revendications 1 ou 2, **caractérisé en ce que** l'alliage de titane et de molybdène présente une teneur en molybdène d'environ 15 %.

4. Procédé selon l'une des revendications précédentes, **caractérisé en ce que** la trempe s'effectue de préférence à l'eau froide.

5. Implant médical constitué d'un alliage de titane et de molybdène β coulé à modèle perdu, dont la teneur en molybdène est de 15 %, le reste étant du titane, et dont la granulométrie moyenne est d'au moins 0,3 mm et le module d'élasticité de 59,4 kN/mm² à 68 kN/mm².

6. Implant selon la revendication 5, **caractérisé en ce qu'**il a été comprimé en conditions isostatiques à chaud et a subi un recuit de solubilisation.

7. Implant selon les revendications 5 ou 6, **caractérisé en ce que** l'alliage de titane ne présente pas de phase ω.

8. Implant selon l'une des revendications 5 à 7, **caractérisé en ce que** l'alliage de titane est exempt de vanadium et d'aluminium.

9. Implant selon l'une des revendications 5 à 8, **caractérisé en ce qu'**il est une prothèse orthopédique.

10. Implant selon la revendication 9, **caractérisé en ce que** la prothèse est une prothèse d'articulation.

11. Implant selon la revendication 10, **caractérisé en ce qu'**il est la pièce de fémur (1) d'une prothèse de l'articulation de la hanche.

12. Implant selon l'une des revendications 5 à 8, **caractérisé en ce qu'**il est un implant dentaire (3).
